# EUROPEAN PATENT SPECIFICATION

(11) **EP 1 487 418 B1**
(45) Date of publication and mention of the grant of the patent: **18.06.2008**
(21) Application number: 03716722.8
(22) Date of filing: 19.03.2003
(51) Int. Cl.: A61K 9/16, A61K 9/20

(54) **CO-BEADLET OF DHA AND ROSEMARY AND METHODS OF USE**
CO-PERLCHEN VON DHA UND ROSMARIN UND VERWENDUNGSVERFAHREN
CO-GRANULE DE DHA, ROMARIN ET LEURS PROCEDES D'UTILISATION

(30) Priority: 28.03.2002 US 368352 P
(43) Date of publication of application: 22.12.2004
(73) Proprietor: Alcon, Inc., 6331 Hünenberg (CH)
(72) Inventor: LANG, John, C., Cedar Hill, TX 75104 (US)
(74) Representative: Moore, Barry
(86) International application number: PCT/US2003/008558
(87) International publication number: WO 2003/082249

(56) References cited:
- EP-A- 0 782 883
- WO-A-01/19383
- WO-A-94/01001
- JP-A- 10 286 079
- US-A- 4 525 306
- US-B1- 6 444 242
- ANDERSEN S: "OMEGA-3 FATTY ACIDS IN FOOD FORTIFICATION" LEATHERHEAD FOOD RA FOOD INDUSTRY JOURNAL, GB, vol. 1, no. 1, 21 March 1998 (1998-03-21), pages 33-40, XP001133851
- PATENT ABSTRACTS OF JAPAN vol. 1998, no. 09, 31 July 1998 (1998-07-31) -& JP 10 101550 A (ASAHI CHEM IND CO LTD; TOYO CAPSULE KK), 21 April 1998 (1998-04-21)

## Description

### BACKGROUND OF THE INVENTION

### 1. Field of the Invention

The present invention relates to the field of nutraceuticals and to the use of dietary supplements to maintain or improve ocular health.

### 2. Description of the Related Art

Dietary supplements are recommended for a variety of reasons including the improvement of vision or prophylaxis of vision loss. An example of dietary supplements useful in improving ocular nutrition and promoting healthy eyes are the ICaps® Dietary Supplements (Alcon Laboratories, Inc., Fort Worth, TX). Dietary supplements are generally consumed in the form of powders, tablets, capsules or gel-caps and comprise a variety of vitamins, minerals, and herbal or other organic constituents. Some dietary supplements are formulated with beadlets, which may function as carriers for the nutritional ingredients and may be blended and compressed into tablets or filled into capsules or gel caps.

Beadlets which contain dietary substances are generally small spheroids of less than about a millimeter in diameter. There are a variety of functions and purposes of beadlets. For example, beadlets may provide for the separate confinement (internal) and segregation from complementary (external) ingredients within the dietary supplement. This type of separation can isolate those components with the potential for interaction or reaction and thereby improve the stability and/or the availability of either entrapped or complementary external ingredients. The ingredients may be combined into a beadlet with a complementary component which facilitates digestion or absorption improving bioavailability. More generally, the food industry has utilized microencapsulation to control availability, flavor, or odor and shelf-life and dosage uniformity stability of acidulants, aromas, nutritional oils and supplements, vitamins, minerals, dietary fibers, or leavening agents, and control of moisture, bacterial growth, and bioburden. The chemical industry has utilized the technology for numerous applications from controlling reaction rates to controlling distribution.

Various beadlet compositions are known and can be obtained from a number of food ingredient or supplement manufacturers, chemical or pharmaceutical manufacturers, specialized manufacturers, biotechnology companies and research institutes; and from independent university laboratories. Particular beadlet compositions for nutritional applications have been the subject of numerous patents including U.S. Patent Nos. 4,254,100 (Keller et al.) and 3,998,753 (Antoshkiw et al.). Methods of beadlet manufacture have been disclosed in U.S. Patent Nos. 4,670,247 (Scialpi) and 3,998,753.

Microencapsulation can be accomplished in one of several manners, from a simple capsular reservoir in which a particle, like an oil droplet, is coated with a thin, generally polymeric, coating to a monolithic matrix in which a final porous, commonly polymeric, structure is generated that can accommodate an active component in its interstices. In some instances, such as the technology to be claimed, it has been necessary to combine the two technologies, providing a coated monolithic active carrier. An example of known microencapsulation is described in WO 94/01001.

Current beadlet compositions used in dietary supplements generally are restricted to the use of inert food-grade ingredients and excipients determined to be safe and effective and complementary to a single nutritional compound. In other instances, when molecules of the same class are refined from a particular source, for example a major component with a minor related constituent, and both compounds produce parallel effects, such molecules may not necessarily be isolated but mixed together in a beadlet. These may be considered pseudo-single-component beadlets, and there are examples in the market place, e.g., Lutrinol® and FloraGLO® beadlets, which are a combination of lutein and zeaxanthin as formulated in Retoxil® Dietary Supplements. Examples of ingredients benefiting from beadlet confinement have included natural vitamins such as Vitamins A, D, E, and K; minerals such as iron and sodium bicarbonate; xanthophylls such as lutein, zeaxanthin, canthaxanthin, and astaxanthin; carotenoids and retinoid alone or in combination, , such as beta-carotene, lycopene, and retinol.

Pending U.S. patent application Serial No. 09/397,472 (the '472 application, herein incorporated by reference) and related foreign applications directed to beadlets containing more than one active ingredient. More specifically, those applications are directed to a beadlet containing one or more xanthophylls, one or more carotenes/retinoids, one or more antioxidants, and one or more solidifying agents. The beadlets of the '472 application are aimed at treating or preventing mammalian diseases or disorders. The '472 application does not discuss the use of DHA or rosemary alone or in any combination.

The preferred form of administering such compositions is the tablet. Unfortunately, previous attempts to create a beadlet containing DHA and produce a tablet from the beadlets have resulted in a tablet with a prominent unpleasant marine odor. The problem of the smell associated with DHA in described in JP-A-7236418 albeit only in the context of a smell correcting agent for the DHA. Further, it was found that the beadlets themselves, after a short period of time, developed an unpleasant marine odor. Thus, what is needed is a beadlet formulation that is both substantially odorless and stable, which does not deteriorate over the shelf life of the product and which can withstand the compressive forces associated with manufacture, and especially of tableting.

### SUMMARY OF THE INVENTION

The present invention overcomes these and other drawbacks of the prior art by providing improved beadlet formulations useful for inclusion in dietary supplements. In particular, the improved beadlets comprise DHA (docosahexaenoic acid); rosemary and/or its components; and excipients. The beadlets are particularly useful for incorporation in dietary supplements customized for improving and maintaining ocular nutrition.

The present invention is also directed to improved dietary supplements comprising the improved beadlets. Preferred dietary supplements have been formulated as an aid to ocular health. The present invention is also directed to methods of using the beadlets and dietary supplements for improving nutritional health. The methods of the present invention are particularly directed to the enhancement of ocular health and the prophylaxis of retinal disorders, including age-related macular degeneration.

The application of the beadlet technology of the present invention to dietary supplements provides, and facilitates development of, enhanced nutritional supplementation. Such technology may aid in increasing bioavailablity of the dietary substances and also provide ease in modifying compositions containing DHA and rosemary within the supplement. Such improvements are believed to be particularly useful in the enhancement of ocular nutrition and improved ocular health.

In certain preferred embodiments, the present invention provides a beadlet and/or its coating containing docosahexaenoic acid (DHA) and/or rosemary. The DHA will be present in the beadlet in a concentration of preferably from about 0.1% to about 50% and the rosemary will be present in the beadlet in a concentration of preferably from about 0.1 % to about 50%. More preferably, the beadlet will contain a concentration of DHA of from about 7% to about 25% and a concentration of rosemary of from about 2% to about 20%. The coating, which serves as a barrier to oxidation from the outside and to escape of odorous agents or their byproducts from the inside, generally consists of a physically or chemically crosslinked or associated polymer and excipients, and may also include an agent such as rosemary to mask odors or prevent chemical dergradation.

It will be understood by the skilled artisan that the percentage ranges above (i.e., 0.1% to 50%, etc.) include all points in between said range. That is, it includes 0.2%, 0.3%, 0.4% and so on, 1.0%, 1.1%, 1.2% and so on, 5.0%, 5.1%, 5.2% ... 5.7%,5.8%,5.9% and so on up to and including 20%, 25%, 30%, 40%, etc.

The beadlets of the invention are virtually odorless. As used herein, the term "odorless" means that the beadlets of the invention have virtually no odor. Depending on the sensitivity of the nose, of course, some may detect no odor, some may detect a slight odor, and some may detect more than a slight odor. The term odorless as used herein is not meant to require that all beadlets are completely without odor but to require that the beadlets are substantially without odor. That is, the odor is substantially diminished compared to beadlets not having the combination of DHA and rosemary of the present invention.

The DHA for use in the beadlets of the present invention may come from fish oil or from fermentation of algae, but could also be derived from flaxseed or soybean or canola oil, borage, eggs or organ meats.. Most preferably, the DHA for use in the beadlets of the present invention is without any significant initial odor, e.g. that from fermentation of algae.

The beadlets of the invention may be in the form of capsular reservoir or monolithic matrix. Capsular reservoirs for an oily active, such as those of the current invention, typically consist of at least one surface-active agent, such as phospholipids and watersoluble polymers, utilized to stabilize microparticles of the active agent(s) suspended in a medium in which they do not dissolve. The coating may be any film-forming type of coating material, such as carbohydrates (acacia and cellulose derivatives and dextrans to gelatin), gluten, polyesters, starch, lactide-glycolide copolymers, waxes, *etc.* One of skill in the art may easily select appropriate coatings based on their properties and their compatibility with the active component(s) and selected/required excipients.

Monolithic matrices essentially trap the active agent(s) within a "web" of polymer. While the monolithic matrix may be formed using any known method, it will generally be formed by precipitation polymerization, coacervation of polymeric blends, condensation polymerization, or by simple drying. In certain embodiments, the core of the carrier may comprise a monolithic matrix while the remainder of the beadlet is a capsular reservoir. In a preferred aspect, the core may be generated with acrylates.

The matrix may be loaded with active agent(s) either before or after polymerization. Whether loading occurs before or after polymerization will depend on the nature of the active agent(s) and/or upon the capacity of the carrier. Such determinations are well within the knowledge and the skill of the ordinary skilled artisan.

In addition, either type of microparticle may require other excipients such as plasticizers, dispersants, colorants and/or opaquants, extenders, and fillers.

Further, where the active agent(s) are unstable it may be further desirable to combine the capsular reservoir and matrix technologies. That is, the active agent(s) may be embeded within a matrix and then the matrix coated to restrict transport of degradizer into the core. This embodiment is preferred where one or more of the active agents are antioxidants. In certain preferred aspects, protective antioxidants can be placed in the core to protect the most vulnerable specie, and may also be placed in the coating. Such a coating can serve two purposes: first, the coating isolates the active and may reduce the rate at which reactive oxygen reaches the active embedded in the core; and second, the antioxidant in the coating serves to reduce the limited amount of oxygen in the head space of the container, generally a plastic bottle of tablets or capsules.

Preferably, the beadlet of the invention will include the following components: two or more active agents; crystallizing or solidifying components, which may include active agents; polymers, either synthetic or biopolymeric; precipitating coacervating components; fillers, which may be inert components, may impart important physical properties to the beadlet, such as hardness or size characteristics, or may provide important chemical characteristics important to one or both of the actives, such as binding or stabilizing characteristics; plasticizers, which prevent the beadlet from being too fragile and crumbling during processing; and chemical agents which protect one or more of the active agents, improving their stability.

The present invention further provides a method of maintaining or improving ocular health in a mammal. The method of the invention generally includes administering to a mammal a composition comprising beadlets, wherein said beadlets comprise DHA and rosemary. In preferred embodiments, the beadlets for use in the methods of the invention will be as described above. Typically, the composition for use in the methods of the invention can be administered in many forms, including powder, capsule, caplet, gel cap or tablet. Most preferably, the composition will be administered in the form of a tablet, a tablet also intended to deliver other micronutrients of value in maintaining ocular nutrition.

### DETAILED DESCRIPTION PREFERRED EMBODIMENTS

The present invention is directed to improved beadlet formulations, improved dietary supplement formulations comprising the improved beadlets and methods of use. As used herein, "dietary supplement(s)" or the shortened form, "supplement(s)," refer to any finished, dietary supplement dosage form containing dietary substances and suitable for ingestion by a host, e.g., human or other mammal.

Docosahexaenoic acid (DHA) is an ω-3. essential fatty acid derived from fish oil or by fermentation, such as from algae. Both minor components of DHA and the by-products of its oxidation have been observed to possess an objectionable aroma, sometimes described as a "marine odor." Producers have attempted to eliminate this drawback of the use of DHA by devising a purer product, by creating carriers capable of protecting the product from oxidation, by masking the odor with a more acceptable fragrance (*e.g*., a citrus fragrance), or a combination of these technologies. For example, both the fermentation process and the isolation of the DHA from the broth have been improved. The result has been a DHA product that has significantly diminished marine odor relative to that obtained from fish oil.

To improve stability of that product and maintain the more odor-free status, the DHA was encapsulated in a beadlet. In one form, the beadlet was produced using gelatin beadlet technology. The beadleted DHA was then formed into a tableted product. When the DHA-containing beadlets were compared with DHA-containing beadlets manufactured using fish-oil derived DHA, a significant improvement in the odor was detected. However, when the beadlets were made into tablets, the marine odor was prevalent, regardless of the source of the DHA. The marine odor was found to increase with time.

Initial studies of tableted formulations of DHA oil indicated that in order to control the dominant marine odor emanating from the tablets, it would be necessary to encapsulate this ω-3 fatty acid. Algal DHA possessed a less offensive odor than that derived from fish oil, and beadlets of the algal DHA were least offensive. However, following tableting, some of the offensive odor was regenerated. An analagous instability had been observed with the compression of lutein beadlets ... only after compression were they observed to be unstable. It is believed that the effect of compression was to expose an increasing fraction of the compound to oxidation. The compression of the DHA beadlets to produce tablets appeared to produce a similar effect; namely, that compression was allowing an increasing fraction of the DHA to be exposed to oxidation, and that it was the oxidized ('rancid') product that generated the more offensive odor. Thus, the present inventor conceived that an antioxidant in sufficient concentration might serve to protect, or assist in protecting, beadletted DHA exposed to the pressure of tablet compression.

It was known that including antioxidants safe for human consumption, such as tocopherols (vitamin E-related compounds) and ascorbates (vitamin C derivatives), in beadlets with lutein protected the lutein from oxidation. Without being bound to any theory, it is believed that the antioxidants protect the lutein by behaving in specific ways: (1) all of the oxygen in the head space eventually reacts with antioxidants; (2) any oxygen which diffuses through the plastic bottle also will react with the confined antioxidants; (3) the oxygen reacts more rapidly with the most oxidizable antioxidants. Thus, the antioxidants in the beadlets are "used up" before oxidization can effect the more important active ingredient. While the DHA has been found to be protected by the rapidly oxidizable components in rosemary, other sources of even more rapidly oxidizable concentrated botanically derived antioxidant are anticipated to provide comparable benefit and are envisioned to be encompassed by the claimed technology.

The present inventor has found that some antioxidants, namely vitamins C and E and rosemary, are effective in protecting retinas from acute light-induced toxicity. Cellular antioxidants function in a cascade of reactions in order to protect sensitive organelles against reactive oxygen species in metabilizing tissues, in which oxygen eventually is reduced to water. For example, ascorbate is known to regenerate the reduced specie of Vitamin E from the oxidized specie. Therefore, the present inventor conceived that rosemary in sufficient quantities in a co-beadlet with DHA might serve multiple purposes. First, it acts as a second active ingredient, providing protection for the eyes and improving ocular health. Second, it acts as a "stabilizer," increasing shelf-life of the product. Finally, it acts as a "de-odorizer," masking the marine-odor of the DHA by preventing its oxidation, and imparting a favorably perceived fragrance.

The present inventor has found that adding rosemary and/or its components to DHA-containing beadlets, such that both "active" ingredients are present in a single beadlet, eliminates the off-odor associated with oxidized DHA while stabilizing the composition, thus increasing its shelf-life. It is contemplated that virtually any beadlet technology, such as that described in U.S. Patent Nos. 4,254,100; 3,998,753; 4,670,247; and 3,998,753 will be useful in the practice of the present invention.

Stability of the beadlet in the present context refers to good containing properties, i.e. the encapsulation protects the active compound(s) from exposure to oxidation and other conditions which may adversely affect the compound or its subsequent availability. The rosemary within the same beadlet as the DHA provides even further protection of the DHA from oxidation and other conditions which may cause the production of the characteristic unpleasant "marine" odor, and contributes to its value as an ocular nutrient.

According to a particular embodiment of the present invention, the microencapsulated DHA/rosemary-containing composition contains 0.1% to 40% of DHA, wherein the particle size of the DHA/rosemary beadlets range from about 100 µm to about 600 µm, though occasional large particles up to 800 µm and small fines of as small as 10 µm may be acceptable as well. The microencapsulated compositions of the present invention are suitable for tablet preparation, hard shell capsule filling and incorporating in different foods. According to a particular embodiment of the present invention, the DHA/rosemary mixtures employed herein may further comprise fillers, excipients or additives. Examples of suitable fillers include starch, pectins, carrageenans, xanthan gums, proteins, polyethylene glycols, cellulose derivatives (e.g., methyl cellulose, hydroxypropyl cellulose and ethyl cellulose) and other polysaccharides.

The present invention is advantageous in that it describes microcapsules, or beadlets, containing DHA and rosemary having virtually no offensive odor, with improved stability, relatively high content of the active agents and improved bioavailability of the active agents. The advantages of the claimed invention derive from the presence of the DHA/rosemary mixtures together in the same beadlet thus decreasing or eliminating offensive odors and increasing stability and shelf-life. The microcapsules of the present invention are tablet grade, i.e., suitable for use in tableting. Preferred compositions of the present invention are gelatin free.

The beadlets of the invention are capable of protecting the active agents (DHA and rosemary) from oxidative damage accompanying exposure to oxygen in the package headspace and/or transported across the package barrier during the shelf-life storage of the product. The beadlets of the invention are further capable of withstanding the compressive forces of 15 SCUor greater and preferably 20 SCU or greater occurring during tableting so that the confined antioxidants of the final product are compromised neither in stability nor in efficacy.

The present invention additionaly provides a composition of the dosage form, containing both the beadlets with the properties defined above as well as other antioxidants themselves (which may or may not be confined in beadlets), such that the most oxidizable component, generally the antioxidants found in rosemary, is present in sufficient abundance to react and deactivate the oxygen in the head space, and protect all of the remaining antioxidants (carotenoids, xanthophylls, vitamins, other polyphenolics, *etc.).* The amount of rosemary in the body of the tablet (not included in the amount contained in / on the carrier beadlet [above]) generally will be greater than 3 mg / tablet and preferably greater than 5 mg / tablet when these tablets are packaged conventionally in bottles of 60 tablets and the container is LDPE or the equivalent in oxygen transmissability, and the protective antioxidant is *unfortified* rosemary oil. As will be well understood by those trained in the art, the amount may be reduced if concentrated forms of the reactive antioxidants in rosemary are utilized. The amount of rosemary in the body of the tablet preserves activity of all the tablet antioxidants, whereas the rosemary contained within the beadlets provides nutritional and therapeutic value, as well as stabilization for the beadlet.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1

### Excipients for gelatin-based beadlets

In the following examples of the technology, actives and coactives refer to DHA and rosemary, or the components thereof. Specific examples of the technology include composition of the beadlets, composition of an optional coating, and ingredients and excipients contained in a complete tablet. Examples of beadlets include:

| | |
|---|---|
| Core Coactives | 15 % |
| Vegetable oil and Oleoresin | 18.5% |
| Hydrolyzed Gelatin | 27.5 % |
| Sucrose | 12 % |
| Ascorbyl Palmitate | 3 % |
| Coating Rosemary (LycoRose) | 3 % |
| Coating Corn Starch and cellulose | 16 % |
| Water | 5 % |

### Example 2

### Excipients for nongelatin-based beadlets

| | |
|---|---|
| Core Coactives: | 24 % |
| Sodium alginate: | 24.8 % |
| Isolated soy protein: | 16.5% |
| Silicon dioxide: | 2 % |
| Coating Hydroxypropyl cellulose (Klucel) | 14.5 % |
| Coating Herbalox type O (rosemary) | 4.8 % |
| Lyc-o-rose (rosemary): | 1.9 % |
| Ethoxylated glycerides: | 4.8 % |
| Sucrose ester: | 1.9 % |
| Water and Ca: | ~5 % |

### Example 3

### Examples of the coating technology

Excipients for a coating of beadlet cores, especially for a non-gelatin based core

| | |
|---|---|
| Active or Coactives | 3% |
| Hydroxypropylcellulose | 16% |
| Methanol | 30% |
| Acetone | 51 % |

A. Excipients for a coating of beadlet cores, especially for a gelatin-based core

| | |
|---|---|
| Active or Coactives | 4% |
| Corn Starch | 10% |
| Cellulosic | 22% |
| Water | 60% |

### Example 4

### Examples of actives in tablet and capsule formulations useful for maintaining ocular health

A. Actives in an ophthalmic formulation

| Ingredient | leaps Rev 3 Formula (per tablet) | Units |
|---|---|---|
| Vitamin A | 2,500 | IU |
| (β-carotene) | 1.5 | Mg |
| Vitamin B-2 | 5 | Mg |
| Folate | 100 | µg |
| Vitamin B-12 | 3 | µg |
| Vitamin C | 200 | Mg |
| Vitamin E | 75 | IU |
| Copper | 0.5 | Mg |
| Manganese | 5 | Mg |
| Selenium | 20 | µg |
| Zinc (acetate) | 7.5 | Mg |
| Lutein (max) | 2 | Mg |
| Zeaxanthin (min) | I | Mg |
| DHA (docosa- | 5 | Mg |
| hexaenoic acid [22:6ω3]) | | |
| Rosemary (minimum) | ≤ 5 | Mg |

B. Actives & excipients (indented) in an ophthalmic formulation:
Ascorbic Acid
   Gelatin
   Hydroxypropyl Methylcellulose
dl-Alpha Tocopherol Acetate
   Dicalcium Phosphate
   Microcrystalline Cellulose
   Magnesium Stearate
   Sucrose
   Silicon Dioxide
Zinc Acetate Dihydrate
Manganese Amino Acid Chelate
   Corn Starch
   Water
   Sodium alginate
Selenium Amino Acid Chelate
   Soy protein (isolated)
   Titanium Dioxide
   Hydroxypropyl cellulose (Klucel)
   Fatty acids (DHA excipients)
DHA (in oil carrier)
Copper Amino Acid Chelate
Riboflavin
   Polyethylene Glycol
Lutein / Zeaxanthin
   Water and Ca
   Ethoxylated glycerides
   Ascorbyl Palmitate
Beta Carotene
Rosemary (from Herbalox type O)
   Sodium Ascorbate
   dl-Alpha Tocopherol
Zeaxanthin
   Canola oil (Herbalox excipient)
   Soybean oil (Herbalox excipient)
   Excipients
   Sorbic Acid
   Polysorbate 80
   Sodium Benzoate
Folic Acid
   Vegetable oil (Lyc-o-Rose excipient)
   Carnauba Wax
Cyanocobalamin

### Example 5

Manufacture of tablets from the microcapsules described in Examples 1 and 2. The ingredients, all core actives and excipients including the cobeadleted DHA and rosemary, are blended and granulated to form a homogenous mass, which is stored in nearly full, low-headspace, sealed plastic bags. As quickly as possible, eliminating any significant delays, the blend is tableted to a hardness of approximately 22 SCU, and the tablets again stored in nearly full, low-headspace, sealed plastic bags until the tablets can be coated, a hiatus that will be kept to a minimum. The tablets are finally spray coated, dried, and packaged.

### References

The following references, to the extent that they provide exemplary procedural or other details supplementary to those set forth herein, are specifically incorporated herein by reference.

**United States Patents** 3,998,753 4,254,100 4,670,247

## Claims

1. A beadlet comprising a microencapulsated composition including docosahexaenoic acid (DHA) and rosemary.

2. The beadlet of claim 1, wherein said beadlet is odorless.

3. The beadlet of claim 1, wherein said DHA is obtained by fermentation from algae.

4. The beadlet of claim 1, further comprising at least one antioxidant other than rosemary.

5. The beadlet of claim 1, wherein the concentration of DHA is from about 0.1 % to about 50% and the concentration of rosema y is from about 0. 1% to about 50%.

6. The beadlet of claim 5, comprising from about 7 % to about 25% DHA and from about 2% to about 20% rosemary.

7. A tablet comprising the beadlet of claim 1.

8. The tablet of claim 7, further comprising as excipients outside of the beadlets, at least 3 mg rosemary oil.

9. The tablet of claim 8, wherein the tablet comprises at least 5 mg rosemary oil.

## Patentansprüche

1. Kügelchen, umfassend eine mikroverkapselte Zusammensetzung, die Docosahexaensäure (DHA) und Rosmarin einschließt.

2. Kügelchen nach Anspruch 1, wobei das Kügelchen geruchlos ist.

3. Kügelchen nach Anspruch 1, wobei die DHA durch Fermentation aus Algen erhalten wird.

4. Kügelchen nach Anspruch 1, weiterhin umfassend mindestens ein anderes Antioxidationsmittel außer Rosmarin.

5. Kügelchen nach Anspruch 1, wobei die Konzentration von DHA von ungefähr 0,1 % bis ungefähr 50% ist und die Konzentration von Rosmarin von ungefähr 0,1 % bis ungefähr 50% ist.

6. Kügelchen nach Anspruch 5, umfassend von ungefähr 7% bis ungefähr 25% DHA und von ungefähr 2% bis ungefähr 20% Rosmarin.

7. Tablette, umfassend das Kügelchen nach Anspruch 1.

8. Tablette nach Anspruch 7, weiterhin umfassend als Hilfsstoffe außerhalb der Co-Kügelchen mindestens 3 mg Rosmarinöl.

9. Tablette nach Anspruch 8, wobei die Tablette mindestens 5 mg Rosmarinöl umfasst.

## Revendications

1. Granules comportant une composition micro-encapsulée incluant de l'acide docosahexaénoïque (DHA) et du romarin.

2. Granules selon la revendication 1, dans lequel ledit granule est inodore.

3. Granules selon la revendication 1, dans lequel ladite DHA est obtenue par fermentation d'algues.

4. Granules selon la revendication 1, comprenant en outre au moins un antioxydant autre que le romarin.

5. Granules selon la revendication 1, dans lequel la concentration en DHA est d'environ 0,1 % à environ 50 %, et la concentration en romarin est d'environ 0,1 % à environ 50 %.

6. Granules selon la revendication 5, comportant d'environ 7 % à environ 25 % de DHA et d'environ 2 % à environ 20 % de romarin.

7. Comprimé comportant les granules de la revendication 1.

8. Comprimé selon la revendication 7, comportant en outre, comme excipients autres que les co-granules, au moins 3 mg d'huile de romarin.

9. Comprimé selon la revendication 8, dans lequel le comprimé comporte au moins 5 mg d'huile de romarin.
